(19) Europäisches Patentamt
European Patent Office
Office européen des brevets

(11) **EP 2 221 608 B1**

(12) **EUROPÄISCHE PATENTSCHRIFT**

(45) Veröffentlichungstag und Bekanntmachung des Hinweises auf die Patenterteilung:
**12.08.2015 Patentblatt 2015/33**

(21) Anmeldenummer: **09153113.7**

(22) Anmeldetag: **18.02.2009**

(51) Int Cl.:
*G01N 21/86* *(2006.01)*          *G01N 21/84* *(2006.01)*
*G01N 21/35* *(2014.01)*          *G01N 35/04* *(2006.01)*

(54) **Testverfahren zur Untersuchung einer Körperflüssigkeit**

Test method for examining a bodily fluid

Procédé de test destiné à examiner un liquide corporel

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(43) Veröffentlichungstag der Anmeldung:
**25.08.2010 Patentblatt 2010/34**

(73) Patentinhaber:
• **F. Hoffmann-La Roche AG**
  **4070 Basel (CH)**
  Benannte Vertragsstaaten:
  **AT BE BG CH CY CZ DK EE ES FI FR GB GR HR HU IE IS IT LI LT LU LV MC MK MT NL NO PL PT RO SE SI SK TR**
• **Roche Diagnostics GmbH**
  **68305 Mannheim (DE)**
  Benannte Vertragsstaaten:
  **DE**

(72) Erfinder:
• **Miltner, Karl**
  **D-67227 Frankenthal (DE)**

• **Lorenz, Robert**
  **D-67547 Worms (DE)**
• **Porsch, Ulrich**
  **D-69469 Weinheim (DE)**
• **Knapp, Clemens**
  **D-58519 Viernheim (DE)**

(74) Vertreter: **Pfiz, Thomas et al**
**Wolf Pfiz & Gauss**
**Patentanwälte**
**Hauptmannsreute 93**
**70193 Stuttgart (DE)**

(56) Entgegenhaltungen:
**EP-A1- 1 785 730          EP-A1- 1 873 515**
**EP-A2- 1 702 565          WO-A1-99/18426**
**WO-A1-2005/032372      DE-A1- 19 811 622**
**JP-A- 11 064 322          JP-B- 7 026 964**
**US-A- 3 526 480          US-A- 4 313 735**
**US-A- 5 051 901          US-A- 5 304 468**
**US-A1- 2006 243 031**

EP 2 221 608 B1

**Beschreibung**

Die Erfindung betrifft ein Testverfahren zur Untersuchung einer Körperflüssigkeit insbesondere zur Blutzuckerbestimmung bei dem ein Testband vorzugsweise in Form einer Bandkassette in einem Testgerät eingesetzt wird, um eine Vielzahl von auf dem Testband bevorrateten analytischen Testfeldern durch Bandtransport sukzessive bereitzustellen, wobei das jeweils bereitgestellte Testfeld durch einen Benutzer mit der Körperflüssigkeit beaufschlagt wird und mittels einer geräteseitigen Messeinheit unter Erfassung von Messsignalen photometrisch abgetastet wird. Die Erfindung betrifft weiter eine entsprechende Testvorrichtung.

Eine gattungsgemäße Testbandvorrichtung ist beispielsweise aus der EP-Anmeldung Nr. 08166955.8 der Anmelder bekannt. Dort ist eine Bandkassette mit einem Testband beschrieben, auf dem sich neben den analytischen Testfeldern auch Positioniermarken befinden, um für jeden betreffenden Bandabschnitt eine zuverlässige Positionierung in verschiedenen Funktionsstellungen zu gewährleisten.

Aus der DE 199 32 846 A1 ist ein Verfahren zur Erkennung der Fehlpositionierung eines optisch auswertbaren Teststreifens bekannt, das auf dem Vergleich zweier Messwerte aus voneinander in Einschubrichtung des Teststreifens in einem Testgerät beabstandeten Messflecken beruht. Die Verhältnisse bei Teststreifensystemen sind allerdings schon insoweit kaum mit Bandsystemen vergleichbar, als die Teststreifen einzeln in eine Geräteführung eingesetzt werden, während der Bandtransport und die Bandführung auf Seite des Verbrauchsmittels erfolgt.

Das Dokument WO2005/032372 offenbart ein Verfahren zur Untersuchung von Körperflüssigkeiten mittels eines Testbands in Form einer Bandkassette, wobei ein Leerwert eines unbenutzten Testfeldes des Testbands mit einem Sollwert verglichen wird. Damit kann eine Verfärbung oder sonstige Veränderung, die auf Unbrauchbarkeit hindeutet, erkannt werden.

Ausgehend hiervon liegt der Erfindung die Aufgabe zugrunde, die im Stand der Technik vorgeschlagenen Testverfahren weiter zu verbessern und eine erhöhte Sicherheit gegen Fehlbedienung und Fehlmessungen zu gewährleisten.

Zur Lösung dieser Aufgabe wird die im unabhängigen Anspruch angegebene Merkmalskombination vorgeschlagen. Vorteilhafte Ausgestaltungen und Weiterbildungen der Erfindung ergeben sich aus den abhängigen Ansprüchen.

Die Erfindung liegt darin, dass ein Chargenkontrollwert auf einem dem Testband zugeordneten Speichermittel gespeichert wird, dass aus einer Leermessung des noch unbenutzten ersten Testfelds ein Testfeldkontrollwert ermittelt wird, und dass die Verwertbarkeit des ersten Testfelds durch Vergleich des Chargenkontrollwerts und des Testfeldkontrollwerts bestimmt wird. Durch einen solchen Qualitätscheck können schädliche Einflüsse auf das als Verbrauchsmittel beispielsweise erst nach längerer Lagerzeit eingesetzte Testmaterial erkannt werden. Als Ergebnis dieser Prüfung kann auch das gesamte Testband als nicht verwertbar eingestuft werden, wenn der Testfeldkontrollwert des ersten Testfelds auf dem Testband um mehr als eine vorgegebene Toleranz von dem Chargenkontrollwert abweicht.

Erfindungsgemäß wird der Chargenkontrollwert im Zuge einer chargenweisen Herstellung von Testbändern durch Vermessen von Testfeldern und Kalibrierfeldern auf dem Testbandmaterial bestimmt. Dies lässt sich aufgrund der homogenen Verarbeitungsprozesse bei Herstellung der Tests in Bandform zuverlässig durchführen.

Um eine tolerierbare Veränderung für nachfolgende Messungen zu berücksichtigen, ist es vorgesehen , dass der Testfeldkontrollwert eines bereitgestellten und als verwertbar eingestuften Testfelds des Testbands als Bandkontrollwert geräteseitig gespeichert wird, und wenn zur Verwertbarkeitsprüfung des nächsten Testfelds dessen entsprechend ermittelter Testfeldkontrollwert mit dem gespeicherten Bandkontrollwert verglichen wird.

Eine vorteilhafte Messwertreferenzierung lässt sich dadurch realisieren, dass durch Erfassung eines dem jeweiligen Testfeld zugeordneten, vorzugsweise weißen Kalibrierfelds mittels der Messeinheit eine Kalibriermessung durchgeführt wird, und dass der Testfeldkontrollwert als Relativwert aus der Leermessung und der Kalibriermessung ermittelt wird.

Für eine weitgehend automatische Verarbeitung ist es auch bei der Erfindung vorgesehen, dass der Chargenkontrollwert in einem auf der Bandkassette aufgebrachten Speichermittel, vorzugsweise in einem RFID-Chip, gespeichert wird, so dass ein geräteseitiger Vergleich ohne weitere Benutzerinteraktion erfolgen kann.

Im Folgenden wird die Erfindung anhand eines in der Zeichnung dargestellten Ausführungsbeispiels näher erläutert. Es zeigen:

Fig. 1    ein analytisches Testbandsystem zur Blutzuckerbestimmung umfassend ein Handgerät und eine Testbandkassette in einer aufgeschnittenen perspektivischen Darstellung;

Fig. 2    eine Ausschnittsvergrößerung der Fig. 1 im Bereich einer Messspitze;

Fig. 3    einen Testbandabschnitt in der Draufsicht;

Fig. 4    ein Messwert-Diagramm in verschiedenen Phasen des Messablaufs;

Fig. 5    ein Messwert-Diagramm in Abhängigkeit der Konzentration eines Analyten für zwei verschiedene Wellenlängen.

EP 2 221 608 B1

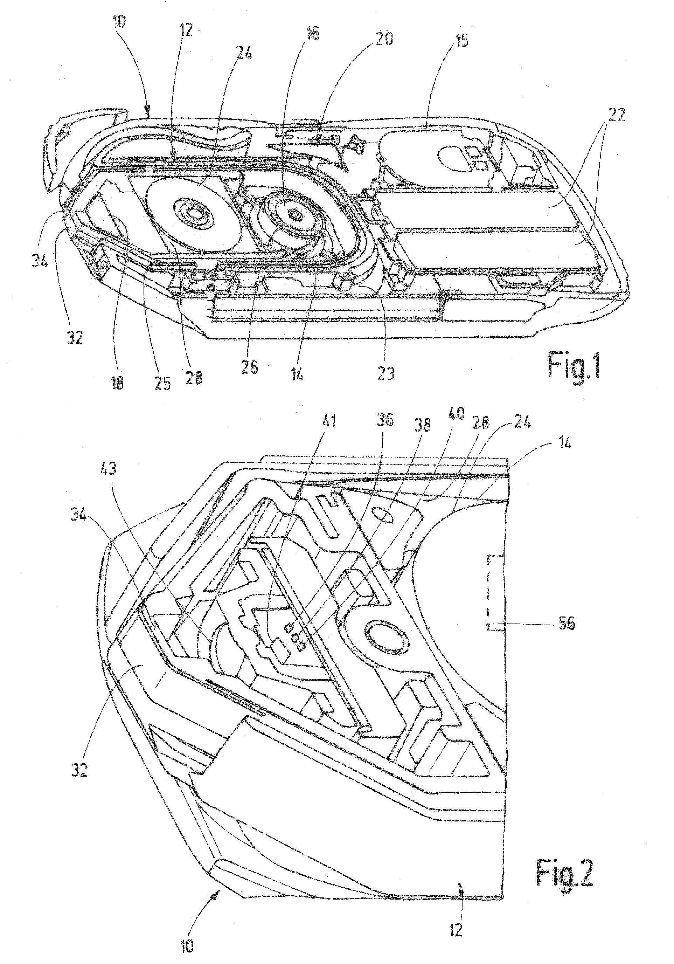

Das in Fig. 1 gezeigte Testbandsystem ermöglicht in einem Handgerät 10 den Einsatz einer Bandkassette 12 mit vorspulbarem Testband 14 als Verbrauchsmittel zur Durchführung von Glukosetests, wobei Funktionsprüfungen in verschiedenen Phasen des Messablaufs vorgenommen werden. Das generelle Geräteprinzip ist in der EP-Anmeldung Nr. 02026242.4 beschrieben, worauf hier Bezug genommen wird.

Das Handgerät 10 besitzt einen Bandantrieb (Motor 15 mit Antriebsspindel 16), eine Messeinheit 18, eine mikroprozessorgestützte Steuereinrichtung 20 und eine Energieversorgung 22. Eine nicht gezeigte Anzeige ermöglicht die Ausgabe von Messergebnissen und Gerätemeldungen für den Benutzer.

Die in ein Aufnahmefach 23 des Geräts 10 einsetzbare Bandkassette 12 umfasst eine Vorratsspule 24 für unverbrauchtes Testband 14 und eine mit dem Antrieb 16 koppelbare Aufwickelspule 26 für verbrauchtes Testband sowie eine Bandführung 25 mit einer Umlenkspitze 34. Die Vorratsspule 24 ist in einer gegenüber der Umgebung abgedichteten Vorratskammer 28 angeordnet.

Das Testband 14 ist abschnittsweise mit Testfeldern 32 versehen, die somit in einer gegebenen Reihenfolge in Bandtransportrichtung gesehen angeordnet sind. Hierbei ist zu berücksichtigen, dass die mit Blut kontaminierten Testfelder 32 auf der Aufwickelspule 26 entsorgt werden und somit ein Rückspulen des Bandes nicht praktikabel ist.

Das jeweils bereitgestellte bzw. aktive Testfeld 32 ist im Bereich der von außen zugänglichen Umlenkspitze 34 vorderseitig mit Probenflüssigkeit, insbesondere Blut oder Gewebeflüssigkeit beaufschlagbar. Der Nachweis des Analyten (Glukose) erfolgt durch rückseitige reflexionsphotometrische Erfassung einer Farbänderung der Testfelder 32 mittels der Messeinheit 18. Zu diesem Zweck sind die Testfelder 32 als Trockenreagenzschicht auf einer transparenten Trägerfolie aufgebracht. Durch entsprechenden Bandvorlauf können die Testfelder 32 sukzessive zum Einsatz gebracht werden. Auf diese Weise lassen sich Vielfachtests für eine Patienten-Selbstkontrolle durchführen, ohne dass Verbrauchsmittel häufig ausgetauscht werden müssten.

Wie in Fig. 2 gezeigt, weist die in die Kassette 12 eingreifende gerätefeste Messeinheit 18 drei Leuchtdioden 36, 38, 40 als Strahlungsquelle sowie eine Photodiode 41 als Detektor für eine reflektionsphotometrische Signalerfassung auf. Eine Optik 43 ermöglicht einen gebündelten Strahlengang unter Abbildung von Leuchtflecken definierter Größe und Intensität auf der Bandrückseite. Die mittlere Leuchtdiode 38 strahlt im sichtbaren (roten) Wellenlängenbereich bei ca. 650 nm, während die äußeren Leuchtdioden 36, 40 im Infrarot bei 875 nm arbeiten. Das auf dem Teststreifen 48 rückwärts gestreute Licht wird mit der Photodiode 41 in einem gegebenen Zeittakt erfasst.

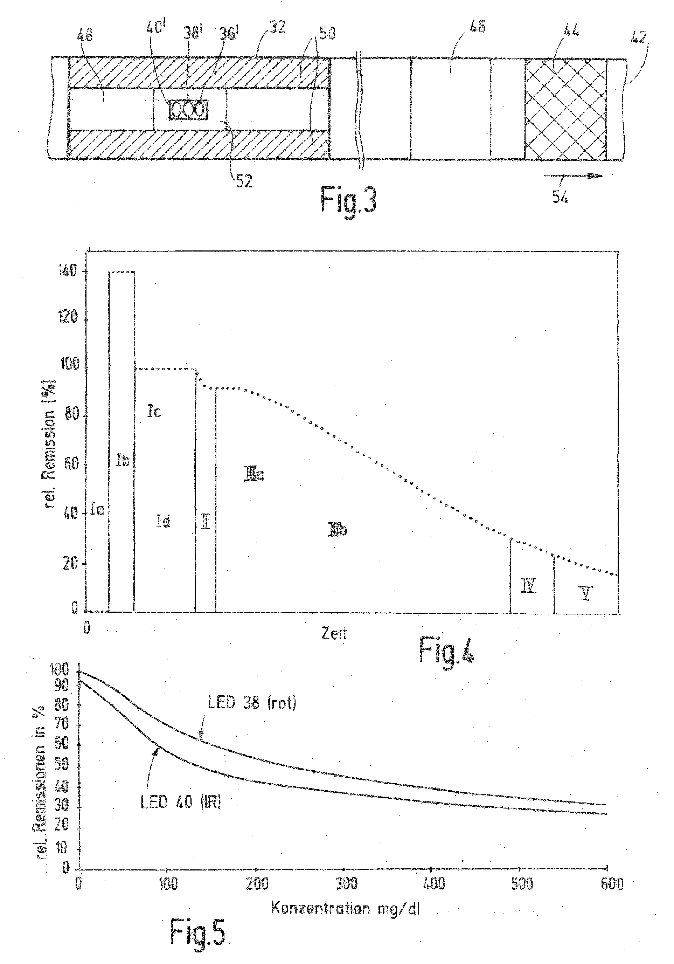

wie in Fig. 3 veranschaulicht, befinden sich die voneinander beabstandeten Testfelder 32 einzeln auf jeweils einem zugeordneten Bandabschnitt 42, der zusätzlich mit weiteren Prüf- bzw. Kontrollfeldern in Form eines Schwarzfelds 44 und eines Weißfelds 46 versehen ist. Das Testfeld 32 weist einen durch die Testchemieschicht gebildeten zentralen Teststreifen 48 auf, der von zwei hydrophoben Randstreifen 50 seitlich begrenzt ist. Die auf dem Testfeld 32 vorderseitig applizierte Probenflüssigkeit benetzt den Teststreifen 48 in Form eines Probenflecks 52, der durch die Leuchtflecken 36', 38', 40' der Leuchtdioden 36,38,40 in der Messposition an der Umlenkspitze 34 von der transparenten Bandrückseite her abgetastet wird. Aufgrund des nur in einer Richtung (Pfeil 54) möglichen Bandtransports werden allerdings zunächst die Prüffelder 44, 46 vor der eigentlichen Messung erfasst, wie nachstehend näher erläutert.

In der Standby-Position befindet sich das Weißfeld 46 des noch unbenutzten Bandabschnitts 42 an der Umlenkspitze 34 vor der Messeinheit 18. Das in weißer Farbe auf das Trägerband 34 aufgedruckte Weißfeld 46 ist so bemessen, dass das von der Messeinheit 18 erfasste Messfenster vollständig überdeckt wird. In gleicher Weise kann auch ein vorgeordnetes Schwarzfeld 44 vor Einnahme der Standby-Position zur Vermessung positioniert werden.

Wie aus Fig. 4 ersichtlich, ist der Messzyklus für jeden Bandabschnitt 42 in verschiedene Phasen unterteilt. In der Phase Ia wird das Schwarzfeld 44 zur Verschmutzungserkennung und ggf. zur geräteseitigen Eigenkorrektur abgetastet, wie es nachstehend noch näher erläutert wird. In Phase Ib erfolgt die Vermessung des Weißfelds 46 zur Bandqualitätsüberprüfung und ggf. zur Eigenkorrektur. Phase Ic sieht die Gewinnung eines so genannten Trockenleerwerts TLW an dem noch unbenutzten Testfeld 32 vor. Sodann ergeht eine Aufforderung zum Blutauftrag an den Benutzer (Id). Die Bereitstellungsphase ist damit abgeschlossen.

In Phase II erfolgt eine Benetzungserkennung an dem Testfeld 32 mittels der IR-Leuchtdioden 36, 40. Bei der Benetzung des Teststreifens 48 findet ein Einbruch der Signalintensität statt.

Anschließend wird die Kinetik des analytspezifischen Messsignals anhand der Farbveränderung des Teststreifens 48 in den Phasen III und IV im Messtakt von z.B. 0,2 s verfolgt. Die Endphase IIIb der Kinetikverfolgung ergibt sich bei Erreichen einer Abbruchschwelle der sich in Abhängigkeit von der chemischen Reaktionsgeschwindigkeit abschwächenden Signaländerung. Sodann wird in Phase IV eine Zweifachmessung mittels LED 38 vorgenommen, um einen gemittelten Endwert EW zu ermitteln. Die Bestimmung der Glukosekonzentration erfolgt dann in relativer Remission, indem der Quotient aus diesem Endwert EW und dem Trockenleerwert TLW gebildet wird (allgemein errechnet sich die relative Remission aus dem Verhältnis des aktuellen Messwerts zum Trockenleerwert). Phase V sieht noch eine Homogenitätsmessung des Probenflecks 52 zur Unterdosiererkennung vor, die auf dem quantitativen Signalvergleich der beiden IR-LEDs 36, 40 beruht. Im Display des Geräts 10 wird schließlich die Glukosekonzentration für den Benutzer angezeigt.

Außer der eigentlichen Messung zur Bestimmung der Glukosekonzentration werden die oben angesprochenen Funktionen bzw. "Fail-Safes" folgendermaßen realisiert:

Die Verschmutzungserkennung erfolgt mit LED 38 nach dem Einlegen der Kassette 12 und im Anschluss an jede Glukosemessung über eine Messung des Signaloffsets auf dem Schwarzfeld 44. Dieser Offset wird von dem gesamten Messumfeld bei eingeschalteten LEDs erzeugt, ohne dass ein Test beteiligt ist. Er geht damit als additive Größe bei der Ermittlung des Messwerts ein. Aufgrund von Verschmutzungen oder sonstigen Optikveränderungen im Strahlengang beispielsweise durch Fremdkörper, Staub und Kratzer wird ausgesendetes Licht teilweise reflektiert und zum Detektor 41 geleitet. Das Schwarzfeld 44 dient bei der Offseterkennung als Ersatz für einen schwarzen Hohlraum, der kein Licht zurückwirft. Grundsätzlich wäre es aber auch möglich, durch das transparente Trägerband hindurch in den dunklen Geräteinnenraum hinein zu messen.

Der erfasste Signaloffset wird mit einem im Gerät 10 gespeicherten Grenzwert verglichen, der bei der Produktherstellung als chargenmittelwert ermittelt wurde. Wird der hinterlegte Grenzwert überschritten, so wird eine Fehlermeldung ausgelöst.

Um die Qualität der als Einwegartikel ggf. nach längerer Lagerzeit eingesetzten Bandkassette 12 zu überprüfen, wird zumindest an dem ersten Weißfeld 46 auf dem Testband 14 ein Referenzwert WF erfasst. Anschließend wird eine potentielle Schädigung der Teststreifenchemie beispielsweise aufgrund von Umwelteinflüssen durch eine entsprechende Veränderung des Trockenleerwerts TLW des ersten Testfelds 32 nachgewiesen. Dazu wird nicht der absolute Remissionswert herangezogen, sondern der auf den Referenzwert WF bezogene relative Remissionswert.

Ein entsprechender Chargenkontrollwert CC wird im Zuge einer der chargenweisen Herstellung von Testbändern 14 durch Vermessen von Testfeldern 32 und Weißfeldern 46 auf dem Testbandmaterial bestimmt. Die Bandherstellung erfolgt in einem Rolle-zu-Rolle-Prozess, der eine solche Kontrollwertzuordnung zu einer weitgehend einheitlichen Beschichtung erlaubt. Der Chargenkontrollwert wird in einem RFID-Chip 56 auf der Kassette 12 gespeichert und durch die Geräteelektronik 20 ausgelesen und verarbeitet. Der RFID-Chip 56 ist außenseitig auf der Kassette 12 aufgebracht und in der aufgeschnittenen Darstellung nach Fig. 2 nur symbolisch gezeigt.

Die Testfeld-Qualitätsprüfung fällt negativ aus, wenn folgende Bedingung erfüllt ist:

$$TLW_1 \: / \: WF_1 \: < \: CC \: - \: \Delta C \qquad (1)$$

wobei $\Delta C$ ein Toleranzwert ist und der Index 1 sich auf den ersten Testbandabschnitt 42 bezieht. In diesem Fall wird eine entsprechende Fehlermeldung ausgegeben und die Kassette 12 ggf. verworfen.

Bei positivem Ergebnis ist es auch möglich, für die nachfolgenden Tests eine Qualitätsprüfung in engeren Schranken durchzuführen. Hierzu wird der relative Remissionswert $C_{n-1}$ des aktuell benutzten Testfelds in einem Gerätespeicher abgespeichert und entsprechend der obenstehenden Gleichung (1) anstelle des Chargenkontrollwerts CC eingesetzt. Eine nachfolgende Qualitätsprüfung eines nächsten Testfelds n fällt also negativ aus, wenn:

$$TLW_n \: / \: WF_n \: < \: C_{n-1} \: - \: \Delta C \qquad (2).$$

Generell wird durch eine herstellerseitige Kalibrierung der Geräte 10 sichergestellt, dass relevante Messwerte nur im spezifizierten Messbereich aller optoelektronischen Komponenten erzeugt werden können. Hierbei erfolgt eine Kalibrierung der elektrischen Parameter der drei LEDs 36, 38, 40 und der optischen Kenngrößen.

Prinzipiell ist auch ein Eigenkorrekturverfahren bzw. eine geräteseitige Kalibrierung möglich, um den spezifischen Einfluss des Signaloffsets und schwankender Absolutmesswerte auf die Messwertbestimmung zu minimieren. Herstellungsbedingt variiert der optische Offset von Kassette zu Kassette. Hinzu kommt, dass aufgrund der Trennung von geräteseitiger Optik und kassettenseitiger Bandführung ein mit Toleranzen behafteter Abstandsanteil auftritt.

Für die Referenzmessung dienen wiederum die Schwarz- und Weißfelder 44, 46, die sich auf dem Testband 14 vor jedem Testfeld 32 befinden. Bereits in der Produktion werden diese Felder vermessen und mit einem Chargenmittelwert versehen. Diese Werte werden als Referenzwert auf dem RFID-Chip 56 abgelegt.

Der nach dem Einlegen einer Kassette 12 an dem ersten Schwarzfeld 44 gemessene Schwarzfeldwert wird überprüft, ob er mit einer vorgegebenen Toleranz am herstellerseitigen Chargenmittelwert für den optischen Offset liegt. Ist dies der Fall, wird der Chargenmittelwert beibehalten. Weicht der gemessene Schwarzfeldwert von diesem Toleranzbereich ab, so wird die Differenz zu dem Chargenmittelwert bestimmt und dem optischen Offset zugerechnet. Der optische Offset wird bei den nachfolgend an den Testfeldern 32 gewonnenen Brutto-Messsignalen subtrahiert.

Die Korrektur des optischen Offsets wird jedoch nur bis zu einem festgelegten Grenzwert durchgeführt. Bei einer Überschreitung dieser Grenze wird wie oben beschrieben eine Fehlermeldung ausgelöst. Vor jedem nachfolgenden Test wird die Schwarzfeldmessung nur zur Verschmutzungserkennung verwendet.

Bei der Weißfeldkalibrierung wird der individuelle Empfindlichkeitswert der Kassette bestimmt, indem der am Weißfeld 46 erfasste Messwert mit dem auf dem RFID-Chip 56 hinterlegten Chargenmittelwert in absoluter Remission verglichen wird. Liegt der gemessene Weißfeldwert $m_K$ innerhalb eines Toleranzbereichs am Chargenmittelwert $m_W$, so wird für eine nachfolgende Skalierung des Offset-korrigierten Brutto-Messsignals der Chargenmittelwert $m_W$, herangezogen und ansonsten die individuelle Kassettenempfindlichkeit $m_K$. Bei der Überschreitung eines Grenzwertes der Abweichung wird jedoch eine Fehlermeldung ausgegeben.

Um eine ungewollte Messwerterzeugung durch Fehlbedienung weitgehend auszuschließen, kann ein Kontrollwert aus einer zeitlichen und/oder wellenlängenabhängigen Änderung der Messsignale ermittelt werden, wobei die Messsignale dann in Abhängigkeit von einem Schwellenwer des Kontrollwerts als gültig weiter verarbeitet oder als fehlerhaft verworfen werden.

Ein erster solcher Fehlerfall kann darin bestehen, dass aufgrund der Abstandsabhängigkeit des Messprinzips durch Andrücken gegen die Umlenkspitze 34 ein Messstart und ein artifizielles Messergebnis erzeugt werden könnte, ohne dass eine Probe appliziert wurde. Um dies ausschließen zu können, werden Messsignale an dem Testfeld 32 bei zwei unterschiedlichen Wellenlängen erfasst, wobei der Kontrollwert aus einer Signaldifferenz der wellenlängenunterschiedlichen Messsignale ermittelt wird.

Wie aus Fig. 5 ersichtlich, ergeben sich bei einer Probenvermessung mit unterschiedlichen Wellenlängen unterschiedliche Werte der relativen Remission über den gesamten Bereich der zu untersuchenden Analyt- bzw. Glukosekonzentration. Dieser Signalunterschied entsteht durch Befeuchten des Testfelds 32 mit der Körperflüssigkeit (deshalb ist auch bei Probenkonzentration 0 bereits ein Unterschied zu finden) und verstärkt sich, wenn das Testchemiesystem verstärkt Reaktionsfarbe bildet. Wird also nur durch Andrücken ein Messsignal erzeugt, kann der typische Unterschied in den beiden wellenlängenunterschiedlichen LEDs 38, 40 durch das fehlende Befeuchten und die fehlende Reaktionsfarbe nicht beobachtet werden, wodurch ein Fehlerfall erkannt wird. Der vorgegebene Schwellenwert des Signalunterschieds kann beispielsweise bei 3% relativer Remission liegen.

Ein weiteres Szenario einer ungewollten Messwerterzeugung besteht darin, dass die fälschliche Erkennung eines Blutauftrags durch eine Bandverschiebung provoziert wird. Wird durch eine Benutzermanipulation ein dunkler Randstreifen 50 des Testfelds 32 in den Strahlengang der Messeinheit 18 verschoben, können hohe Messwerte erzeugt werden, ohne dass Probe aufgetragen wurde.

Typische Reaktionskinetiken von Blutproben auf einem Testfeld 32 zeigen jedoch oberhalb von ca. 100 mg/dl Glukosekonzentration einen Signalhub von ca. 10%, wie er als Differenz der ersten und letzten Kinetikmessung in Phase III (Fig. 4) ermittelt wird. Wird hingegen das Testband in Phase II wie oben beschrieben bloß verschoben, erfolgt ein schlagartiges Verdunkeln und danach ein konstantes Signal - es wird also in Phase III keine veränderliche Reaktionskinetik und kein nennenswerter Signalhub beobachtet. Somit ist eine Fehlererkennung dadurch möglich, dass der Kontrollwert aus einer Signaldifferenz von am Anfang und Ende eines Messintervalls erfassten Messsignalen ermittelt wird, und dass bei einer Signaldifferenz nahe Null ein Fehlerfall erkannt wird.

Befindet sich ein Testfeld 32 vor der Optik 43 im Zustand der Probenauftragserkennung (Phase II in Fig. 4), so interpretiert die Steuereinrichtung 20 des Geräts 10 eine Signaländerung um ein festgelegtes Maß als Probenauftrag und beginnt mit der Auswertung. Sowohl hohe Luftfeuchtigkeit als auch Sonneneinstrahlung können unter ungünstigen Umständen ohne Probenauftrag bereits zu einer solchen Signaländerung und damit zu einem Messstart führen.

Um dies zu verhindern, wird der zeitliche Verlauf der Leersignaländerung im Zustand der Probenerwartung geprüft. Während das Auftragen einer Blutprobe bereits innerhalb einer halben Sekunde einen Remissionsabfall von mehreren Prozent erzeugt, wird ein solcher Abfall bei Einwirkung von Sonnenlicht oder Luftfeuchte erst über einen Zeitraum von mehr als 20 Sekunden erreicht. Demzufolge ist es möglich, dass an dem für die Probenapplikation bereitgestellten Testfeld zyklisch Leerwerte erfasst werden, und dass der Kontrollwert aus einer Leerwertänderung gegenüber einem anfänglichen Leerwert ermittelt wird, wobei bei einer Leerwertänderung über einem vorgegebenen Schwellenwert (von beispielsweise ca. 5%) eine Flüssigkeitsapplikation und unterhalb davon ggf. nach einer gegebenen Wartezeit ein Fehlerfall erkannt wird.

Ein weiteres Messproblem kann darin bestehen, das sich der Trockenleerwert eines bereitgestellten, aber noch unbenutzten Testfelds 32 beispielsweise durch Licht- oder Feuchtigkeitseinfluss verändert und damit als Bezugsgröße für die Bestimmung der relativen Remission zu einer Verfälschung führt. Der Messwert des unbenutzten Testfelds kann daher im Zustand der Probenerwartung zyklisch überprüft und entweder nachgeführt werden, um somit Messwertverfälschungen zu verhindern, oder ab einem bestimmten Grenzwert beispielsweise von mehr als 0,5%/s relativer Remissionsänderung die Messung mit einer Fehlermeldung abzubrechen.

**Patentansprüche**

**1.** Testverfahren zur Untersuchung einer Körperflüssigkeit insbesondere zur Blutzuckerbestimmung bei dem ein Testband (14) in Form einer Bandkassette (12) in einem Testgerät (10) eingesetzt wird, um eine Vielzahl von auf dem Testband (14) bevorrateten analytischen Testfeldern (32) durch Bandtransport sukzessive bereitzustellen, wobei das jeweils bereitgestellte Testfeld durch einen Benutzer mit der Körperflüssigkeit beaufschlagt wird und mittels einer geräteseitigen Messeinheit (18) photometrisch abgetastet wird, **dadurch gekennzeichnet, dass** ein Chargenkontrollwert im Zuge einer chargenweisen Herstellung von Testbändern durch Vermessen von Testfeldern (32) auf dem Testbandmaterial bestimmt wird, dass der Chargenkontrollwert auf einem dem Testband (14) zugeordneten, auf der Bandkassette (12) aufgebrachten Speichermittel (56) gespeichert wird, dass aus einer Leermessung des noch unbenutzten ersten Testfelds ein Testfeldkontrollwert ermittelt und mit dem Chargenkontrollwert verglichen wird, dass der Testfeldkontrollwert eines bereitgestellten und als Ergebnis des Vergleichs des Chargenkontrollwerts und des Testfeldkontrollwerts als verwertbar eingestuften Testfelds des Testbands (14) als Bandkontrollwert geräteseitig gespeichert wird, und dass zur Verwertbarkeitsprüfung des nächsten Testfelds (32) dessen entsprechend ermittelter Testfeldkontrollwert mit dem gespeicherten Bandkontrollwert verglichen wird.

**2.** Testverfahren nach Anspruch 1, **dadurch gekennzeichnet, dass** das erste Testfeld (32) oder das gesamte Testband (14) als nicht verwertbar eingestuft wird, wenn der Testfeldkontrollwert des ersten Testfelds auf dem Testband (14) um mehr als eine vorgegebene Toleranz von dem Chargenkontrollwert abweicht.

**3.** Testverfahren nach Anspruch 1 oder 2, **dadurch gekennzeichnet, dass** durch Erfassung eines dem jeweiligen Testfeld (32) zugeordneten, vorzugsweise weißen Kalibrierfelds (46) mittels der Messeinheit (18) eine Kalibriermessung durchgeführt wird, und dass der Testfeldkontrollwert als Relativwert aus der Leermessung und der Kalibriermessung ermittelt wird.

**4.** Testverfahren nach einem der Ansprüche 1 bis 3, **dadurch gekennzeichnet, dass** der Chargenkontrollwert in einem RFID-Chip gespeichert wird.

## Claims

**1.** Test method for analysing a body fluid in particular for blood sugar determination in which a test tape (14) in the form of a tape cassette (12) is used in a test device (10) in order to successively provide a plurality of analytical test fields (32) stored on the test tape (14) by means of tape transport, wherein the body fluid is applied by a user to the test field provided at a time and the said test field is photometrically scanned using a measuring unit (18) of the device, **characterized in that** a batch control value is determined during a batchwise production of test tapes by measuring of test fields (32) on the test tape material, that the batch control value is stored on a storage medium (56) which is assigned to the test tape (14) and applied to the tape cassette (12), that a test field control value is determined from a blank measurement of the yet unused first test field and compared with the batch control value, that the test field control value of a test field of the test tape (14) that has been provided and classified as usable as a result of the comparison of the batch control value and the test field control value is stored on the device as a tape control value, and that for a check of the usability of the next test field (32) the test field control value thereof which is correspondingly determined is compared with the stored tape control value.

**2.** Test method according to claim 1, **characterized in that** the first test field (32) or the entire test tape (14) is classified as being not usable when the test field control value of the first test field on the test tape (14) deviates by more than a specific tolerance from the batch control value.

**3.** Test method according to claim 1 or 2, **characterized in that** a calibration measurement is carried out by detecting a preferably white calibration field (46) assigned to the respective test field (32) by means of the measuring unit (18), and that the test field control value is determined as a relative value from the blank measurement and the calibration measurement.

**4.** Test method according to one of the claims 1 to 3, **characterized in that** the batch control value is stored in an RFID-chip.

## Revendications

**1.** Procédé de test destiné à l'analyse d'un fluide corporel, notamment à la détermination de la glycémie, dans lequel une bande de test (14) en forme de cassette à bande (12) est insérée dans un appareil de test (10), afin de mettre à disposition de manière successive, par le biais du transport de la bande, une pluralité de champs de test analytiques (32) stockés sur la bande de test (14), le champ de test respectivement mis à disposition étant exposé par un utilisateur au fluide corporel et soumis à un balayage photométrique au moyen d'une unité de mesure (18) côté appareil, **caractérisé en ce qu'**une valeur de contrôle de lot est déterminée, dans le contexte d'une fabrication de bandes d'essai par lots, en mesurant des champs de test (32) sur le matériau de bande de test, **en ce que** la valeur de contrôle de lot est enregistrée sur un moyen d'enregistrement (56) associé à la bande de test (14), appliqué sur la cassette à bande (12), **en ce qu'**une valeur de contrôle de champ de test est déterminée à partir d'une mesure à vide du premier champ de test encore inutilisé et est comparée à la valeur de contrôle de lot, **en ce que** la valeur de contrôle de champ de test d'un champ de test de la bande de test (14) mis à disposition et considéré comme utilisable suite au résultat de la comparaison de la valeur de contrôle de lot et de la valeur de contrôle de champ de test est enregistrée, côté appareil, en tant que valeur de contrôle de bande, et **en ce que**, pour vérifier l'aptitude à l'utilisation du champ de test (32) suivant, la valeur de contrôle de champ de test de ce dernier, déterminée de manière correspondante, est comparée à la valeur de contrôle de bande enregistrée.

2. Procédé de test selon la revendication 1, **caractérisé en ce que** le premier champ de test (32) ou l'ensemble de la bande de test (14) est considéré comme non utilisable lorsque la valeur de contrôle de champ de test du premier champ de test sur la bande de test (14) s'écarte de la valeur de contrôle de lot d'une valeur dépassant une tolérance prédéfinie.

3. Procédé de test selon la revendication 1 ou 2, **caractérisé en ce qu'**une mesure de calibration est réalisée au moyen de l'unité de mesure (18) qui détecte un champ de calibration (46), de préférence blanc, associé au champ de test (32) respectif, et **en ce que** la valeur de contrôle de champ de test est déterminée en tant que valeur relative à partir de la mesure à vide et de la mesure de calibration.

4. Procédé de test selon l'une des revendications 1 à 3, **caractérisé en ce que** la valeur de contrôle de lot est enregistrée dans une puce RFID.

7

Fig.1

Fig.2

8

Fig.3

Fig.4

Fig.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 08166955 A
- DE 19932846 A1
- WO 2005032372 A
- EP 02026242 A